(19) **Europäisches Patentamt**

**European Patent Office**

**Office européen des brevets**

(11) **EP 1 070 036 B1**

(12) **EUROPEAN PATENT SPECIFICATION**

(45) Date of publication and mention
of the grant of the patent:
**18.06.2003 Bulletin 2003/25**

(51) Int Cl.$^7$: **C07C 29/136**, C07C 31/20

(21) Application number: **99919189.3**

(86) International application number:
**PCT/EP99/02297**

(22) Date of filing: **02.04.1999**

(87) International publication number:
**WO 99/052845 (21.10.1999 Gazette 1999/42)**

(54) **PROCESS FOR THE PRODUCTION OF BUTANEDIOL**

VERFAHREN ZUR HERSTELLUNG VON 1,4-BUTANDIOL

PROCEDE DE PRODUCTION DE BUTANEDIOL

(84) Designated Contracting States:
**AT BE CH DE ES FR GB IT LI NL**

(30) Priority: **09.04.1998 BE 9800276**

(43) Date of publication of application:
**24.01.2001 Bulletin 2001/04**

(73) Proprietor: **BASF AKTIENGESELLSCHAFT**
**67056 Ludwigshafen (DE)**

(72) Inventors:
• **BERTOLA, Aldo**
**I-20121 Milano (IT)**

• **RANGHINO, Giovanni**
**I-20090 San Felice-Segrate (IT)**

(74) Representative: **Sarpi, Maurizio**
**Studio FERRARIO**
**Via Collina, 36**
**00187 Roma (IT)**

(56) References cited:
**EP-A- 0 722 923** **US-A- 4 584 419**

**Description**

The present invention relates to a process for the production of 1,4-butanediol by vapour phase flow catalytic hydrogenation of gamma-butyrolactone, succinic or maleic anhydride methyl esters, or mixtures thereof, wherein the hydrogen flow is enriched with CO, so as to accomplish an extra production of synthesis methanol, with a consequent increase in the yield of the final product.

It is known from the prior art that there exist many processes for the preparation of butanediol using dicarboxylic acid esters with four carbon atoms as starting materials.

In USSR Patent N°400,567 a copper chromite catalysed liquid phase conversion of esters into BDO at temperatures ranging between 280 and 300°C and 300 bar pressure is given.

In US Patent N°4,613,707 a conversion of diethylsuccinate into BDO at 200°C T and 135 bar pressure with a mainly copper and aluminium borate catalyst is given.

In US Patent N°2,079,414 a vapour phase hydrogenation of esters is given, on a copper chromite type catalyst and temperatures ranging between 300 and 400°C.

In US Patent n° 2,040,944 a broad description of both liquid and vapour phase ester hydrogenations is given, using copper chromite catalysts. In one example, a liquid phase butyl succinate hydrogenation at 207 bars is described.

In US Patents n°'s 4,584,419 and 4,751,334, assigned to Davy Mc Kee Ltd. (U.K.) processes for the large scale production of BDO are described and they involve four carbon dicarboxylic acid ester hydrogenations.

The operating conditions of the hydrogenations claimed in the patents assigned to Davy McKee Ltd. are pressures ranging between 25 and 75 bars, and temperatures between 150° and 240 °C, in the presence of a stabilised cuprous chromite type catalyst in its reduced form.

Moreover in the latter patent it is stated that the process which is claimed in it yields a 79.3% conversion into the desired product, after the first stage.

There is no indication of the overall conversion, but it is not at all unfair to be sure that it is somewhat lower than the above value.

Likewise, a 78.3% overall conversion is obtained in the process claimed in the former patent.

Clearly, as the above values hold on an industrial scale, they leave much room for substantial improvements.

In EP -0722 923 it is described a process for the hydrogenation of maleic acid, maleic anhydride or other hydrogenatable precursor to 1,4-butanediol, wherein a hydrogenation catalyst consisting of palladium, silver and rhenium on a carbon support, is used. Said known process is characterized by high yields of 1,4-butanediol, but the noble metal catalyst described is a powerful, hydrogenation catalyst completely inadequate to produce methanol, because of its costs, and never used in the production of methanol.

Aim of the present invention is therefore to propose a process which allows much higher conversion rates, therefore increases the profitability of its application to the plant scale.

Another aim of the present invention is that of proposing a methodology for the contemporaneous production of BDO and Methanol (MOH) that operates in specific conditions and on suitable catalysts, which are employed in the commercial production of methanol.

The above aims are accomplished by a process for the production of 1,4-Butanediol by vapour phase selective hydrogenation of gamma-butyrolactone, maleic or succinic anhydride methyl esters or mixtures of gamma-butyrolactone with the esters thereof, characterised by the fact that synthesis methanol is coproduced by feeding hydrogen gas enriched with carbon monoxide to the reaction.

According to the process of the present invention, methyl dicarboxylic acid esters with four carbon atoms, e. g. dimethylmaleate (DMM) or dimethylsuccinate (DMS), gamma butyrolactone (GBL), or mixtures of GBL with the esters thereof, are utilised as starting materials for the production of BDO.

Methanol synthesis is accomplished by feeding carbon monoxide together with hydrogen gas. The reaction by which BDO forms thanks to the hydrogenation of an ester like DMS for example, is given by the following equation:

$$(A) \quad CH_3\text{-}O\text{-}CO\text{-}CH_2\text{-}CH_2\text{-}CO\text{-}O\text{-}CH_3 + 4H_2 \rightarrow 2CH_3OH + OH(CH_2)_4OH$$

The MOH synthesis reaction is given by the following equation:

$$(B) \quad CO + 2H_2 \rightarrow CH_3OH$$

MOH that forms in reaction (A) is recovered to be fed to an esterification unit .

However from a practical viewpoint, part of the MOH is lost either literally or in the form of by-products. MOH real losses, about 0.1 to 0.3 mols for each mol of BDO produced, may even be compensated by MOH formation according to reaction (B), this making the production unit completely self-sufficient in terms of MOH.

The most important aspect is the neat improvement given by the process object of the present invention in so far as the yield of reaction (A) is concerned, more than its self-sufficiency in terms of MOH consumption, the latter being nevertheless a not unimportant factor.

Surprisingly, the MOH synthesised which adds up to the MOH produced in reaction (A) is not of any hindrance to the BDO synthesis reaction as it would be expected to be. Instead it results to have a remarkably positive effect on the conversion of the ester and/or GBL of the shot.

The surprising observation stated above may be explained by dint of the protective action exerted onto the active sites of the catalyst by the methanol synthesis.

It may be significant that the catalyst employed in the hydrogenation reaction is of either the exact same or similar type as that employed in methanol synthesis, e.g. of the copper-zinc oxide, or cuprous chromite type, this ensuring a compatibility and ease of coexistence of the two reactons in the same vessel.

In the process which is object of the present invention, the typical operating conditions for this reaction are as follows:

| | |
|---|---|
| Reaction type: | adiabatic |
| Molar ratio between $H_2$ and ester: | from 200 to 800, preferably from 200 to 500 |
| Molar ratio between CO and shot ester: | from 0.1 to 1.0 preferably from 0.2 to 0.4 |
| Operating pressure: | from 75 to 120 bars, preferably from 80 to 100 bars |
| Operating temperature: | from 170 to 230°C preferably from 190° to 210 °C |
| Type of catalyst: | Copper/Zinc oxide or cuprous chromite |
| Liquid Hourly Space Velocity: | from 0.1 to 1.0 $hr^{-1}$, and averagely from 0,3 to 0.5 $hr^{-1}$ |

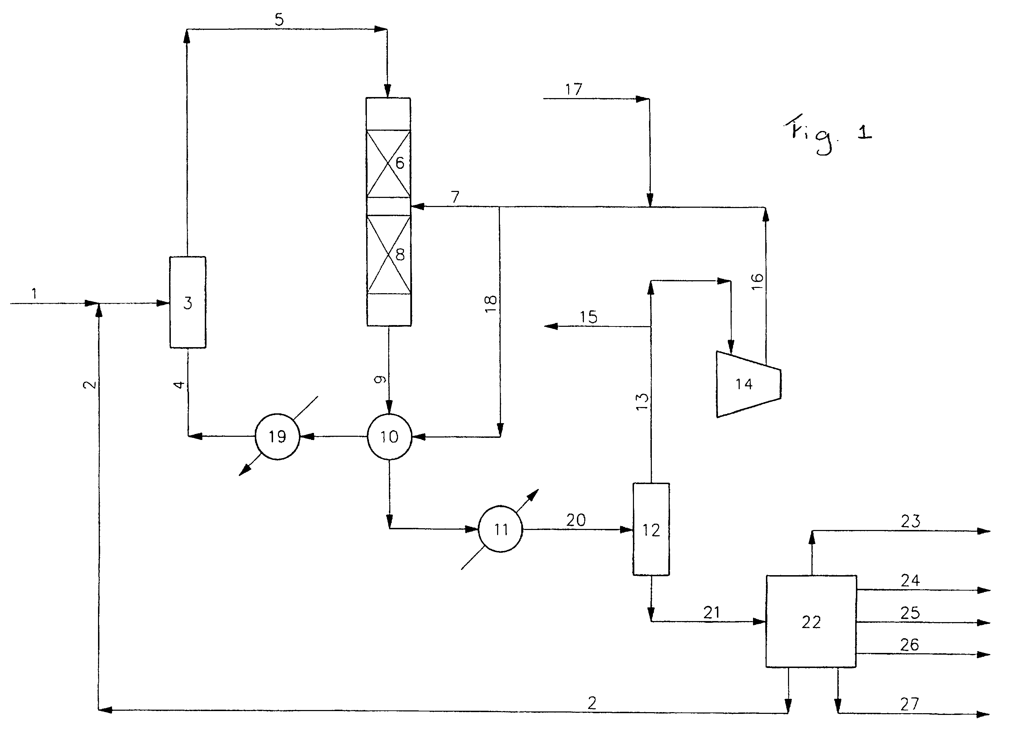

These and other features will be more readily apparent from the following description of a preferred not limiting embodiment of the invention with reference to the accompanying drawing in which a scheme (Fig. 1) of the production process is shown.

The following operating conditions refer to a starting material made up of a mixture of GBL and DMS in 70: 30 proportions by weight.

The feed mixture (Line 1) is fed to vapouriser 3, together with a liquid recycle mixture (Line 2), that contains GBL from fractionation unit 22. In vapouriser 3 both the feed (Line 1) and the recycle (Line 2) come to contact with a hot hydrogen stream (Line 4), and they vapourise. In the gas stream coming out of the vapouriser (Line 5) the molar ratio between hydrogen and ester is 300:1, the molar ratio between CO and ester is 0.2, temperature is 190°C and pressure is 80 ATE.

Such stream feeds Stage I of reaction 6 where there is a copper-zinc oxide type catalyst with a surface area never any smaller than 40 $m^2g^{-1}$.

At the inlet of Stage II in reaction 7, temperature is taken down to approx. 190°C by injection of a cold hydrogen stream (Line 7). Overall Liquid Hourly Space Velocity in the two reaction stages is 0.35 $hr^{-1}$.

At the inlet of Stage II in reaction 8, DMS conversion results to be higher than 98%, while GBL conversion in the feed is higher than 91%.

The overall average conversion value works out to be as high as 93%.

By-products obtained in the process consist of tetrahydrofuran (THF)-about 6% on a molar base with respect to the BDO produced.

Selectivity of the reaction is approx. 99% with respect to the overall amount of BDO and THF produced.

The high molar ratio between hydrogen and carbon monoxide favours the occurrence of methanol synthesis with stepwise conversion values higher than 90%.

The effluent from the reactor (Line 9) cools down in exchanger 10, letting heat to the recycle hydrogen rich stream, and in exchanger 11. Eventually it feeds (Line 20) separator 12 where the condensed organic phase separates from the hydrogen rich gaseous phase.

The gaseous phase coming out of separator 12 (Line 13) is compressed by compressor 14 to be recycled into the reaction system.

A small fraction of the recycle gas is purged (Line 15) to minimise the accumulation of inert materials.

The compressed gas (Line 16) and the feed hydrogen and carbon monoxide (Line 17), partly (Line 7) blend with the effluents from the first stage of reaction 6, and partly (Line 18) pre-heat in exchanger 10 and in terminal heater 19, to subsequently feed (Line 4) vapouriser 3.

The liquid phase coming out of separator 12 feeds (Line 21) product fractionation unit 22, where THF (Line 23), MOH (Line 24), water and light organic by-products (Line 25), an unconverted GBL rich organic fraction (Line 2) bound to be recycled for hydrogenation, heavy organic by-products (Line 26) and BDO (Line 27) separate.

The innovative aspect of the process object of the present invention is better emphasised by a comparison of its performances of which above with those obtained carrying out the hydrogenation with the same shot and operating conditions, nevertheless without streaming any carbon monoxide gas.

It was found that without carbon monoxide, hence without any MOH synthesis, keeping the same high selectivity values, the overall conversion of GBL to DMS decreases from the 93% mark, obtained when feeding carbon monoxide, to approx. 84 %.

The lower conversion rates entice higher investment and production costs, both in the hydrogenation section, and in the subsequent distillation sections where the unconverted fractions bound to be recycled for hydrogenation are separated.

## Claims

1. A process for the production of 1,4-butanediol by vapour phase selective hydrogenation of gamma-butyrolactone, methyl esters of maleic or succinic acid or mixtures of gamma-butyrolactone with the esters thereof, **characterised by** the fact that synthesis methanol is coproduced by feeding hydrogen gas enriched with carbon monoxide to the reaction.

2. A process according to claim 1, **characterised in that** the molar ratio between carbon monoxide and shot ester ranges between 0.1 and 1.0

3. A process according to claims 1 and 2, **characterised in that** the hydrogen to shot ester molar ratio ranges between 200 and 800.

4. A process according to claims 1,2 and 3, **characterised in that** its operating pressure ranges between 75 and 100 bars, and its operating temperature ranges between 170° and 230°C.

5. A process according to claims 1,2,3 and 4, **characterised in that** the hydrogenation catalyst is of the copper-zinc oxide or stabilised cuprous chromite type,with surface area never any smaller than 40 $m^2g^{-1}$.

6. A process according to claim 5, **characterised in that** the vapour phase mixture containing shot hydrogen and ester contacts the catalyst with a liquid hourly space velocity ranging between 0.1 and 1.0 $hr^{-1}$.

## Patentansprüche

1. Ein Verfahren zur Herstellung von 1,4-Butandiol durch selektive Gasphasenhydrierung von gamma-Butyrolacton, Methylestern von Malein- oder Bernsteinsäure oder Mischungen von gamma-Butyrolacton mit Estern davon, **dadurch gekennzeichnet, dass** durch Zuführen von mit Kohlenmonoxid angereichertem Wasserstoffgas in die Reaktion zusätzlich Synthesemethanol hergestellt wird.

2. Verfahren nach Anspruch 1, **dadurch gekennzeichnet, dass** das molare Verhältnis zwischen Kohlenmonoxid und zugeführtem Ester zwischen 0,1 und 1,0 liegt.

3. Verfahren nach Anspruch 1 oder 2, **dadurch gekennzeichnet, dass** das molare Verhältnis zwischen Wasserstoff und zugeführtem Ester zwischen 200 und 800 liegt.

4. Verfahren nach einem der Ansprüche 1 bis 3, **dadurch gekennzeichnet, dass** der Arbeitsdruck zwischen 75 und 100 bar und die Arbeitstemperatur zwischen 170 und 230 °C liegt.

5. Verfahren nach einem der Ansprüche 1 bis 4, **dadurch gekennzeichnet, dass** der Hydrierkatalysator vom Kupfer-Zinkoxid-Typ oder vom Typ stabilisiertes Kupferchromat mit einer Oberfläche von mindestens 40 $m^2g^{-1}$ ist.

6. Verfahren nach Anspruch 5, **dadurch gekennzeichnet, dass** die Gasphasenmischung enthaltend zugeführten Wasserstoff und Ester mit einer flüssigen stündlichen Raumgeschwindigkeit zwischen 0.1 und 1.0 $h^{-1}$ mit dem Katalysator in Kontakt kommt.

**EP 1 070 036 B1**

**Revendications**

1.  Procédé pour la production de 1,4-butanediol par hydrogénation sélective en phase vapeur de gamma-butyrolactone, d'esters méthyliques de l'acide maléique ou succinique ou de mélanges de gamma-butyrolactone avec ces esters, **caractérisé en ce que** du méthanol de synthèse est produit conjointement en introduisant dans la réaction de l'hydrogène gazeux enrichi en monoxyde de carbone.

2.  Procédé selon la revendication 1, **caractérisé en ce que** le rapport molaire entre le monoxyde de carbone et l'ester de la charge est compris entre 0,1 et 1,0.

3.  Procédé selon les revendications 1 et 2, **caractérisé en ce que** le rapport molaire entre l'hydrogène et l'ester de la charge est compris entre 200 et 800.

4.  Procédé selon les revendications 1, 2 et 3, **caractérisé en ce que** sa pression de travail est comprise entre 75 et 100 bars et sa température de travail est comprise entre 170°C et 230°C.

5.  Procédé selon les revendications 1, 2, 3 et 4, **caractérisé en ce que** le catalyseur d'hydrogénation est du type oxyde de zinc-cuivre ou chromite cuivreux stabilisé dont la surface spécifique n'est jamais inférieure à 40 $m^2g^{-1}$.

6.  Procédé selon la revendication 5, **caractérisé en ce que** le mélange en phase vapeur contenant l'hydrogène et l'ester de la charge entre en contact avec le catalyseur à une vitesse spatiale horaire du liquide comprise entre 0,1 et 1,0 $h^{-1}$.

Fig. 1